# EUROPEAN PATENT APPLICATION

(11) **EP 4 623 889 A1**
(43) Date of publication of application: **01.10.2025**
(21) Application number: 24167664.2
(22) Date of filing: 28.03.2024
(51) Int. Cl.: A61K 8/02, A61K 8/04, A61K 8/96, A61Q 19/00, A61Q 19/10

(54) **A SKINCARE COMPOSITION COMPRISING SEDIMENTARY ROCK OPOKA PARTICLES**

(71) Applicant: UAB "IGA LT", 99303 Pagegiai (LT)
(72) Inventor: Gudaviciene, Irina, LT-04125 Vilnius (LT); Gudavicius, Paulius, London, SE100T (GB); Gudaviciute, Julija, London, IG11OZP (GB)
(74) Representative: Zaboliene, Reda

(57) **Abstract**

This invention presents a skincare composition utilizing sedimentary rock Opoka particles, offering a versatile approach to cosmetic formulations. By combining Opoka particles sourced from rock formations or clay deposits with a cosmetically acceptable carrier, this pioneering invention unveils a transformative skincare range. This range includes liquid and solid soap, hand washing paste, and a face mask, all enriched with sedimentary Opoka rock, which is abundant in calcium carbonate and amorphous silica. Opoka's mineral blend embodies cleansing prowess, gentle exfoliation, hydration, and anti-inflammatory properties. The heart of this innovation lies in the meticulous preparation of an aqueous suspension prepared from sedimentary rock Opoka particles, spotlighting its delicate exfoliating nature, purifying capabilities, and profound hydration benefits. The integration process seamlessly combines aqueous Opoka suspensions, both in formulations with cosmetically acceptable carrier, like plant oils and others, and in products where Opoka powder stands alone, laying the foundation for skincare that aims to revitalize, nourish, and cleanse the skin. This method for producing skincare composition is designed for non-therapeutic skincare purposes, offering various customization possibilities for skincare products.

## Description

### TECHNICAL FIELD

The invention belongs to the field of cosmetics, specifically involving a solid and liquid soap, hand washing paste, and face mask containing sedimentary rock Opoka.

### DESCRIPTION OF THE RELATED ART

The skin, as the body's frontline defence, plays a crucial role in shielding our internal systems, while cosmetics contribute significantly to enhancing its appearance and condition. Over the past decade, the cosmetic industry has evolved significantly to meet changing consumer demands. Amidst these changes, cosmetics play an integral role in daily human routines, addressing not only aesthetic desires but also contributing to self-care and confidence.

The growing need for innovative cosmetic solutions reflects an increasing demand for products that not only enhance appearance but also promote skin health and well-being. This demand has driven the industry to explore new ingredients and methods to enhance modern cosmetic formulations. While a variety of cosmetic products cater to specific skin concerns like dryness, elasticity, redness, and cleanliness, among others, certain challenges persist or might be limited in addressing specific skin conditions or concerns.

This invention stands as a response to these challenges, aiming to redefine skincare routines by integrating the remarkable attributes of sedimentary rock Opoka. Beyond just utilizing Opoka in cosmetics, the method of its preparation is equally significant in enhancing its efficacy and suitability for products designed for skin care purposes. Leveraging Opoka's unique attributes and its mineral-rich nature, this innovation aims to create an effective skincare solution that not only deeply cleanses and addresses skin dryness and dehydration but also tackles various other existing skin concerns, surpassing the capabilities of existing skincare products.

The patent document NZ573986A describes a skincare composition for cleaning and cosmetic purposes, consisting of finely ground particles of zeolitic rocks. The products detailed in the document, which contain natural zeolite particles, encompass soaps and similar skincare items designed for cleansing and detoxification. These zeolitic rock particles have a low hardness, ensuring they are gentle on the skin. Various forms of silicon dioxide (including amorphous (opaline) silica, cristobalite, and tridymite) constitute essential components of the products outlined in this document. These compositions within hygiene products exhibit potent detoxifying properties, effectively trapping impurities, toxins, and free metals, and aiding in their removal from the body. However, while both zeolitic rocks and Opoka contain amorphous (opaline) silica, the difference lies in their overall mineral composition. Zeolitic rocks, despite their similar silica content, tend to possess a higher hardness, potentially leading to a harsher abrasiveness on the skin. On the other hand, Opoka, with its dominance of amorphous (opaline) silica, stands out for offering a more delicate texture, ensuring gentler abrasion, and showcasing exceptional conditioning and moisture-retaining abilities beneficial for skin health.

The soothing face mask described in patent document KR20100001199A contains mineral clay powder and is easily removed from the face by hand. The composition of this mask includes mineral clay (which can be kaolin, montmorillonite, illite, and chlorite) powders, sodium alginate, calcium carbonate, magnesium carbonate, and sodium pyrophosphate. The mixture is combined with water and applied to the face in gel form. The mask is left on the face for 10-30 minutes, during which time the skin absorbs the soothing properties of the mineral clay, resulting in a beautiful and revitalized complexion. The mineral clay is finely ground into specific particle sizes to ensure optimal effectiveness. Calcium and magnesium carbonates are included in the composition as sources of divalent ions to improve the overall aggregation of the mask. This aggregation is crucial for the consistency and texture of the mask, ensuring it adheres well to the skin and can be easily removed after use. This innovative product can benefit the skin affected by various environmental factors and is suitable for use in skincare procedures. However, the following invention lacks the Opoka's natural synergy effect stemming from its unique mineral composition, notably its predominant content of calcium carbonate and silica dioxide, offering exceptional skincare benefits like superior cleansing, conditioning, and unmatched moisture retention.

Accordingly, it is desired to leverage sedimentary rock Opoka's unique mineral composition in pioneering a new line of cosmetics, aiming to revolutionize skincare benefits.

This description provides a method for formulating cosmetics utilizing the unique mineral composition of Opoka sedimentary rock. The approach involves integrating Opoka's properties into various skincare products, aiming to harness its potential benefits in enhancing skin health. By infusing Opoka into liquid or solid soaps, hand washing pastes, or face masks, this process seeks to explore its potential impact on skin cleansing, elasticity, tone, sebum control, and redness reduction, ultimately aiming to create innovative skincare solutions harnessing Opoka's mineral-rich nature.

### SUMMARY OF THE INVENTION

The invention is a skincare composition comprising sedimentary rock Opoka particles. The skincare composition additionally comprises a cosmetically acceptable carrier, which may include essential or plant oils, Potassium or Sodium hydroxide, and other ingredients. The skincare composition is prepared as an aqueous suspension mixed with water or as a powder product which is further used for the preparation of final cosmetic or hygiene product.

The Opoka particles used in this composition are obtained from rock formations or clay deposits, and using both raw materials can yield equivalent results. The Opoka dry matter concentration in the suspension ranges from 10 to 80% (w/w). The particle size is in the range from 1 to 209 µm, enhances the effectiveness of the skincare product in the composition.

The skincare composition is available in various forms, such as solid or liquid soap, hand washing paste, or facial mask, catering to different skincare needs.

The method for producing this skincare composition involves preparing an aqueous suspension from Opoka particles and fractionating it through sedimentation. For the aqueous suspension, the Opoka dry matter concentration can be adjusted within the range of 10 to 80% (w/w). The particle size of Opoka particles is in the range from 1 to 209 µm.

The method for producing skincare composition involves using sedimentary rock Opoka particles obtained from either rock formations or clay deposits. If the Opoka is sourced from rock formations, an additional step of mechanical fragmentation is involved to break down the rock into fine particles. The method additionally includes mixing the composition with a carrier that is suitable for cosmetic use, adding further customization additives. Additionally, the Opoka suspension is dried for the preparation of Opoka powder.

The composition is used for the preparation of cosmetic and hygienic products. The invention provides a non-therapeutic method for caring for the skin comprising applying the skincare composition to the skin.

### DETAILED DESCRIPTION OF THE INVENTION

The proposed invention represents a significant improvement at the intersection of skincare innovation and natural mineral utilization, introducing a pioneering approach to cosmetic formulations by harnessing the unique attributes of sedimentary rock Opoka.

Opoka is a sedimentary rock dominated by amorphous (opaline) silica. Over 50 years ago, in the southwestern part of Lithuania, in the Silute district, the Stoniškiai Opoka deposit was excavated. The quarry's resources amount to 15.9 million tons. The valuable layer of the Stoniškiai deposit consists of irregularly stratified Opoka, carbonate Opoka, and siliceous marl. These rocks are relatively lightweight, with a bulk density of 1.74 t/m3. The primary chemical composition of Opoka is presented in Table 1.

**Table 1. The chemical composition of sedimentary rock Opoka**

| Sedimentary Rock | Average values, % | | | | | |
|---|---|---|---|---|---|---|
| | SiO₂ | Al₂O₃ | Fe₂O₃ | CaCO₃ | MgO | SO₃ |
| Opoka | 63,5-73,2 | 1,0-2,1 | 0,3-1,0 | 6,0-17,3 | 0,4-0,5 | 0,2-0,4 |

Opoka is primarily composed of calcium carbonate and amorphous silica, along with traces of magnesium and iron compounds. Calcium (Ca) and silicon (Si) are the two main minerals found in Opoka rock. These two elements offer numerous benefits to the skin, including:
- Cleansing: Amorphous silica, a major component of Opoka, exhibits remarkable sorptive properties due to its porous structure and high surface area. It acts as an efficient adsorbent, attracting and binding impurities, excess oils, and environmental pollutants on the skin's surface. Additionally, its finely textured nature serves as a gentle exfoliant, aiding in the removal of dead skin cells and promoting skin renewal. This dual action of adsorption and exfoliation effectively purifies the skin, unclogs pores, and promotes a clearer complexion.
- Softening and Moisturizing: Calcium and silicon present in Opoka contribute to the skin's hydration and softness by their hydrophilic nature. Their affinity for water molecules facilitates the retention of moisture in the epidermal layers. This hydrating effect helps fortify the skin's natural moisture barrier, preventing transepidermal water loss (TEWL). The resultant increased skin hydration reduces dryness, diminishes the sensation of itchiness, and alleviates flaking, thereby enhancing the skin's overall suppleness and comfort.
- Anti-inflammatory Properties: Amorphous silica within Opoka exhibits notable anti-inflammatory characteristics. It operates by modulating the inflammatory response pathways, specifically by downregulating the expression of pro-inflammatory cytokines. This action suppresses inflammatory cascades, thereby mitigating redness, swelling, and discomfort associated with inflammatory skin conditions like eczema and psoriasis. Additionally, the antiseptic nature of silica aids in inhibiting microbial growth, further contributing to a reduction in inflammation and promoting a healthier skin environment.
- Improved Elasticity: Calcium and silicon play crucial roles in supporting the skin's structural integrity by aiding in the synthesis and maintenance of extracellular matrix components like collagen and elastin. Collagen provides structural support, while elastin confers elasticity and resilience to the skin. The presence of calcium and silicon enhances these processes, stimulating collagen and elastin production. As a result, the skin experiences improved firmness, elasticity, and a reduction in the appearance of fine lines and wrinkles, promoting a more youthful and toned complexion.

The object of this invention was the dusty fraction of Opoka, which forms during mining activities and as a result of natural erosion processes. Within the quarry area, this material settles at the bottom of an accumulation reservoir in the form of Opoka clay. The accumulation reservoir is a part of the quarry drainage system.

The preparation of sedimentary rock Opoka consists of the following stages:
Method 1:
   1. Mechanical fragmentation:
      - The process initiates with the mechanical fragmentation of the rock, involving the breaking down of the solid mass into smaller particles;
   2. Aqueous suspension formation:
      - The resulting rock powder from the fragmentation process is meticulously mixed with water. This meticulous blending aims to create an aqueous suspension that contains 25-35% of Opoka's dry matter. The mixture's consistency and uniformity are paramount during this phase to attain the desired concentration;
   3. Fractionation through sedimentation:
      - Subsequently, the suspension undergoes a fractionation process using sedimentation principles. Sedimentation involves settling the suspended particles under specific conditions. The sedimentation conditions are governed by maintaining a sedimentation bath layer thickness of 0.5-1.0 meters and a duration of 60-120 minutes. During this phase, the heavier particles settle to the bottom of the suspension due to gravity, creating distinct layers based on particle size and density.
Method 2:
   1. Utilization of the dusty fraction formed during quarrying operations:
      - This material settles at the bottom of the accumulation reservoir in the form of Opoka clay. The accumulation reservoir is part of the quarry drainage system;
   2. Aqueous suspension formation:
      - The process involves dispersing Opoka clay in water, aiming to achieve an aqueous suspension with a 25-35% concentration of Opoka's dry matter within the suspension;
   3. Fractionation through sedimentation:
      - Fractionation of rock particles through sedimentation, akin to the process in Method 1, employing the same sedimentation conditions.

The preparation of sedimentary rock Opoka involves the utilization of two distinct methods, both considered optimal for the production of an entire range of skincare products. The selection between these methods is contingent upon the existing reserves of Opoka. It is noteworthy that, in this invention, Opoka clay is chosen due to its current availability; however, equivalent results can be achieved by employing sedimentary rock Opoka formations in the process.

After performing laser diffraction analysis of the Opoka aqueous suspension, the granulometric composition of the suspension (see Table 2) was determined.

**Table 2. Granulometric composition of the Opoka suspension**

| Particle fraction size, µm | Fraction portion, vol.% |
|---|---|
| 0,5-2,2 | 3,5 |
| 2,2-23 | 73,2 |
| 23-60 | 18,3 |
| 60-120 | 3,6 |
| 120-210 | 1,4 |

The fractionated Opoka suspension obtained through sedimentation is poured into closed containers and stored until use.

This Opoka suspension can further be used for the production of various cosmetic products, like solid or liquid soap, hand washing paste or facial mask.

Preparation of Opoka for solid or liquid soap, hand washing paste production:
- The settled Opoka suspension in containers is homogenized with water until a suspension is formed containing about 45-50% dry matter of Opoka.

Preparation of Opoka for facial mask production:
- The settled Opoka suspension in containers is dried and heated at a temperature of +250 °C for 60 minutes.
- The dry residue of Opoka is mechanically crushed into powder.

A thorough microelement analysis of Opoka clay was conducted. The analysis was performed by the accredited laboratory of UAB "SEKARGAS HAMILTON", specializing in the examination of cosmetic products and the raw materials used in their production. Upon the evaluation of the analysis results, it was established that the concentration of heavy metals in Opoka clay did not exceed the specified threshold values set in Germany and Canada for certain heavy metals. After conducting a more extensive analysis of the legal regulations regarding the permissible concentrations of heavy metals in cosmetic products, it was determined that as of today, only Germany and Canada have regulations in place for heavy metals in cosmetics.

Concurrently, analogous research was conducted with the blue clay found in Lithuania, which, according to the data from the conducted literature analysis, is utilized in the production of certain cosmetic products (e.g., facial masks). From the data presented in Table 3, it is evident that the concentration of heavy metals in blue clay is significantly higher than that in Opoka clay and, in most indicators, exceeds the legally established threshold concentrations of heavy metals in Germany (since 2017).

**Table 3. Maximum Permissible Concentrations (MPCs) of Heavy Metals in Cosmetic Products**

| Analyte | MPCs of metals, mg/kg (Germany, Canada) | MPCs of metals, mg/kg (Germany since 2017) | Opoka Sample, mg/kg | Blue Clay Sample, mg/kg |
|---|---|---|---|---|
| Cadmium (Cd) | 5 and 3 | 0.1 | Not found | 0.37 |
| Nickel (Ni) | 10 | 10 | < 0.002 | 414 |
| Lead (Pb) | 2 | 2 | Not found | 18.3 |
| Arsenic (As) | 5 and 3 | 0.5 (2.5 for makeup) | Not found | 427 |
| Mercury (Hg) | 1 | 0.1 | Not found | 0.0022 |
| Antimony (Sb) | 5-10 | 0.5 | Not found | 1.13 |
| Selenium (Se) | n.d. (not determined) | n.d. (not determined) | Not found | 5.1 |

In 2023, a study was commissioned at the laboratory of UAB "SEKARGAS HAMILTON" to investigate the impact of Opoka clay on biomechanical skin parameters and consumers with skin conditions.

The study utilized washed, heated, and finely ground Opoka clay in powder form. Simultaneously, a similar study was conducted using blue clay powder.

Opoka and blue clay powders were used in facial mask procedures 2-3 times per week during a 21-day research period. Summarizing the research findings, specialists from "SEKARGAS HAMILTON" provided the following conclusions:
Opoka Clay:
   - Improves skin elasticity (average 6%) and skin firmness (average 11%).
   - Evens out the skin tone (average 6%).
   - Reduces sebum secretion from sebaceous glands (average 13%).
   - Reduces haemoglobin levels after 21 days of use (average 4%), meaning it reduces visible skin redness.
Blue Clay:
   - Slightly evens out the skin tone (average 1%).
   - Slightly reduces haemoglobin levels after 21 days of use (average 1%), meaning it reduces visible skin redness.
   - As evident from the research results, Opoka clay exhibits high activity in promoting human skin health.

### Examples

The following formulations are provided as illustrative examples of the invention. It's important to note that the invention encompasses various other potential formulations, and these examples are not intended to restrict its scope.

### Example 1: Natural Handmade Solid Soap

In this example, soap is fashioned from a meticulous process involving the blending of plant oils, and sedimentary rock Opoka suspension with sodium hydroxide and distilled water, followed by controlled heating and an infusion of essential oils.

**Table 4. Component Distribution for Natural Handmade Soap**

| Component | Content, wt. % |
|---|---|
| Plant oils | 43-55 |
| Distilled water | 24-27 |
| Sedimentary Rock Opoka Suspension | 5-20 |
| Sodium Hydroxide (NaOH) | 8-11 |
| Essential Oil | Up to 2 |

The oils are carefully measured and poured into the soap production container, where they are thoroughly mixed using an installed mixer. The measured amount of sedimentary rock Opoka water suspension is weighed. The suspension is poured into the soap production container. The mixture in the container is stirred until a homogeneous mixture is formed. The mixture in the soap production container is heated to a set temperature.

Water is thoroughly measured and poured into a stainless-steel container to prepare the alkaline solution. The calculated amount of sodium hydroxide (NaOH) is weighed and uniformly dosed into the container preparing the alkaline solution with water. It is stirred until the caustic soda granules are completely dissolved. When reacting with water, NaOH releases a lot of heat, so the container for preparing the alkaline solution is cooled with water.

The oil and rock mixture is combined with the NaOH solution. The mixture is thoroughly stirred in the container. After all the ingredients have reacted, the essential oil is slowly poured in to ensure thorough mixing and distribution throughout the soap mixture.

The soap mass is stirred until it begins to thicken and acquire a "thick milk" consistency. Upon reaching this stage, the soap mixture is poured into the moulding form. The soap is removed from the moulds and cut into smaller pieces. The cut soap pieces are placed in ventilated boxes and matured in well-ventilated spaces for 30 days to complete the saponification process.

The pH indicator of the matured soap should be 8-9.

### Example 2: Natural Liquid Soap

In this example, liquid soap is made from a precise amalgamation of plant oils, potassium hydroxide, distilled water, sedimentary rock Opoka suspension, and essential oils, followed by controlled heating to facilitate the saponification process.

**Table 5. Component Distribution for Liquid Soap**

| Component | Content, wt. % |
|---|---|
| Plant oils | 39-47 |
| Distilled water | 37-41 |
| Sedimentary Rock Opoka Suspension | 5-10 |
| Potassium hydroxide (KOH) | 8-9 |
| Essential Oil | Up to 2 |

The oils are weighed and then placed in a stainless steel or enamelled pot and mixed. Water is poured into a heat-resistant plastic or stainless-steel container. Potassium hydroxide (KOH) is weighed and added to the prepared container with water. It is stirred with a plastic paddle to ensure complete dissolution of KOH granules. Upon reacting with water, KOH releases a significant amount of heat, thus cooled water is used for the solution.

The oil mixture is then combined with the KOH solution and vigorously stirred. The soap mass is stirred for 4-5 minutes (depending on quantity) until it starts to thicken and acquires a 'condensed milk' consistency. Once this stage is reached, the mixture is set aside for 20 minutes, during which the fatty fraction separates and forms a curd-like mass. It is then stirred again. This process is repeated thrice until the soap mixture attains a thick, uniform consistency.

The container with the soap mixture is placed in a hot water bath (a larger container with heated water) and covered. The soap mass is heated for 3-4 hours until it passes the gel stage. The mixture is regularly stirred to ensure an even saponification process. The resulting mixture is transparent with a soft consistency. The pH is evaluated, with the normal range being 8-9.

The measured quantity of sedimentary rock Opoka suspension is poured in. The mass is thoroughly stirred and left for 12 hours. After the specified time, essential oils are poured into the prepared soap mixture. An additional measured quantity of oil is also added. The soap mass is thoroughly stirred to evenly distribute all components. The mixture is diluted with distilled water at a ratio of 1:3. The finished product is poured into a container.

### Example 3: Natural Hand Washing Paste

This example is a natural hand washing paste which composition consists of plant oils, potassium hydroxide (KOH), distilled water, sedimentary rock Opoka suspension, glycerine, and essential oils.

**Table 6. Component Distribution for Natural Hand Washing Paste**

| Component | Content, wt. % |
|---|---|
| Plant oils | 10-39 |
| Distilled water | 42-45 |
| Sedimentary Rock Opoka Suspension | 5-10 |
| Potassium hydroxide (KOH) | 8-9 |
| Glycerine | 0,5-2,0 |
| Essential Oil | Up to 1 |

The oils are precisely measured and introduced into a stainless steel or enamelled container. Simultaneously, water is carefully placed into a heat-resistant plastic vessel. The measured quantity of KOH is meticulously added to the water-filled container and thoroughly mixed until the KOH granules are completely dissolved. Due to its reaction with water, KOH generates notable heat, necessitating the use of cooled water for the solution. The oil mixture is then blended with the KOH solution and vigorously stirred.

The soap mixture is stirred for 5 minutes (duration varying with quantity), resulting in thickening and achieving a 'condensed milk' consistency. After reaching this stage, the mixture undergoes a 20-minute resting period during which it separates into a fatty fraction and a mass resembling curd. Stirring is resumed, and this process is iterated three times to ensure the formation of a uniform and dense soap mixture.

The container holding the soap mixture is placed in a hot water bath (a larger container filled with heated water) and covered with a lid. The soap mass is heated for 3-4 hours until it passes through the gel stage. Regular stirring during this phase ensures uniform saponification. The resultant mixture appears transparent with a soft consistency. Subsequently, the pH is assessed, aiming for a range between 8 and 9.

Following this assessment, the measured volume of sedimentary rock Opoka suspension is introduced, thoroughly blended, and left to stand for 12 hours. Glycerine is then weighed and mixed into the mixture. Essential oil is incorporated, and the soap mass undergoes further stirring to ensure even distribution of all components. Dilution with distilled water is conducted until the mixture achieves a paste-like texture before transferring the final product into a container.

### Example 4: Facial Mask

In this example, facial mask is meticulously crafted from the fractionated aqueous Opoka suspension.

The fractionated aqueous Opoka suspension is poured into a sealed plastic container with a capacity of 250 litres for storage.

The Opoka suspension is stored in containers until it achieves a condensed state with a moisture content of 45-80%. Subsequently, it undergoes drying and heating in an electric convection oven at a temperature of +250°C for 60 minutes.

The resulting dry Opoka residue is mechanically pulverized into powder using a high-speed grinder. The bulk density of the Opoka powder for facial mask production ranges from 1.0 to 0.5 g/cm³.

The produced powder is packaged in aluminium containers for sale. The further process involves placing a desired quantity of powder into a ceramic or glass vessel. A minimal amount of water is then added and stirred to achieve a uniform mixture. For those with dry skin, the inclusion of a few drops of unrefined oil is an option.

The application of this mask is typically on freshly cleansed skin, while carefully avoiding the area under the eyes. The duration of the mask's presence on the skin varies from 5 to 10 minutes, depending on the individual's skin type. It is crucial to avoid allowing the mask to dry completely and to remove it with warm water. For the best results, a frequency of 2-3 times per week is suggested. The efficacy of the mask can be enhanced by the addition of a few drops of vitamin E.

This facial mask can also be used as a face/neck wash. The skin is first dampened before applying the prepared clay. It is then massaged gently in circular motions and rinsed off with warm water.

In the case of clay bath treatments, the entire contents of the jar can be poured into the bath water and stirred thoroughly to integrate.

## Claims

1. A skincare composition comprising sedimentary rock Opoka particles.

2. The skincare composition according to claim 1, wherein the composition is Opoka aqueous suspension.

3. The skincare composition according to claim 1, wherein the composition is in powder form.

4. The skin care composition according to any of claims 1-3, wherein the composition optionally comprises cosmetically acceptable carrier.

5. The skincare composition according to claim 1, 2 or 4, wherein the concentration of Opoka dry matter in the Opoka suspension is from 5 to 80 % (w/w).

6. The skincare composition according to any of claims 1-5, wherein the sedimentary rock Opoka particles are derived from either rock formations or clay deposits.

7. The skincare composition according to any of claims 1-6, wherein the size of the Opoka particles is from 0,5 to 210 µm.

8. A method for producing skincare composition, comprising preparation of an aqueous suspension from sedimentary rock Opoka particles; and fractionating of Opoka suspension through sedimentation.

9. The method for producing skincare composition according to claim 8, wherein the sedimentary rock Opoka particles are derived from either rock formations or clay deposits.

10. The method for producing skincare composition according to claim 8 or 9, wherein said method additionally comprises mechanical fragmentation when utilizing sedimentary rock Opoka formations.

11. The method for producing skincare composition according to any of claims 8-10, wherein said method optionally comprises mixing of the composition with cosmetically acceptable carrier.

12. The method for producing skincare composition according to any of claims 8-11, wherein said method additionally comprises drying of Opoka suspension.

13. The method for producing skincare composition according to any of claims 8-12, wherein the aqueous suspension includes sedimentary rock Opoka dry matter, which concentration is from 5 to 80 % (w/w).

14. The method for producing skincare composition according to any of claims 8-13, wherein the size of the Opoka particles is from 0,5 to 210 µm.

15. A non-therapeutic method for caring for the skin, comprising applying the composition according to any of claims 1-7 to the skin.
